# EUROPEAN PATENT APPLICATION

(11) **EP 0 818 538 A1**
(43) Date of publication of application: **14.01.1998**
(21) Application number: 96201890.9
(22) Date of filing: 08.07.1996
(51) Int. Cl.: C12N 15/56, C12N 9/42, C07K 16/40, C12N 1/21, A01N 63/00, A23L 1/00, A23L 2/00, C12G 1/00, D21H 17/00, C02F 3/00

(54) **Novel cellulases**

(71) Applicant: RIJKSLANDBOUWUNIVERSITEIT WAGENINGEN, 6700 HB Wageningen (NL)
(72) Inventor: Schots, Arjen, NL-6706 EV Wageningen (NL); Bakker, Jacob, NL-6704 PD Wageningen (NL); Helder, Johannes, NL-3582 AC Utrecht (NL); Gommers, Frederik Jan, Nl-6705 CS Wageningen (NL); Stiekema, Wilhelmus Johannes, NL-6708 BG Wageningen (NL); Roosien, Jan, NL-6714 BS Ede (NL); Goverse, Aska, NL-6703 BS Wageningen (NL); Schouten, Alexander, NL-6866 DE Heelsum (NL); Smant,Geert, NL-6708 CV Wageningen (NL); De Boer, Jan Marinus, Ames, Iowa 500010 (US); Stokkermans, Jacobus Petrus Wilhelmus, NL-5049 JN Tilburg (NL)
(74) Representative: de Bruijn, Leendert C.

(57) **Abstract**

The invention provides peptides having cellulase activity and exhibiting at least 40% amino acid identity in the primary structure with the amino acid sequence shown in one of SEQ ID NO.'s 1, 2, 3 and 4 or comprising a series of at least 7 contiguous amino acids of the amino acid sequence shown in one of SEQ ID NO.'s 1, 2, 3 and 4. Said peptide corresponds to a nematodal peptide especially of a sedentary nematode (such as a cyst nematode or root-knot nematode) or part thereof. Antibodies against such peptides are valuable crop-protecting agents. Furthermore provided are nucleotide sequences encoding these peptides and expression systems comprising at least such a nucleotide sequence. The enzymic peptides are useful in the protection of plants against parasitic nematodes, and in the food and beverage industry, in paper or clothing industry or in waste treatment.

## Description

### Field of the invention

The invention relates to cellulases of a novel class, to their amino acid sequences, and to nucleotides encoding them. The invention also concerns the use of these enzymes in agriculture and in industry and the use of peptides or other compounds inhibiting these enzymes in the protection of crops against plagues.

### Background

Plant parasitic nematodes are an important threat to agriculture because they feed on roots of hosts plant and reduce the possibilities of the plant to take up nutrients from the soil. Depending on how they feed they can be divided in feeding types: 1. sedentary parasites (have swollen females); 2. migratory endoparasites (crawl in the root); 3. semi-endoparasites; 4. ectoparasites, and 5. epidermal and root hair feeders

All plant parasitic nematodes feed by penetrating one or more root cells with their stylet. Endoparasitic nematodes either disrupt cells or move in between cells. For these actions more than muscle force is likely to be required.

Plant parasitic nematodes comprise:
1. Sedentary nematodes; these include:
   a. Cyst nematodes (Heteroderidae family)
   b. Root knot nematodes (Meloidogynidae family)
   c. genus *Nacobbus*;
2. Migratory endoparasites
   d. Pratylenchidae family (genera *Pratylenchus* and *Radopholus* including the burrowing nematode *R. similis* which attacks citrus and other (sub)tropical crops)
   e. Anguinidae family (several members)
   f. Aphelenchoididae family (including the genera *Aphelenchoides* (leaf nematodes), *Bursaphelenchus* and *Rhadinaphelenchus*);
3. Semi-endoparasites
   g. Hoplolaimidae family (spiral nematodes including the genera *Rotylenchus* and *Helicotylenchus*)
   h. genera *Tylenchulus* (including the citrus nematode *T. semipenetrans*) and *Rotylenchulus*;
4. Ectoparasites
   i. Hemicycliophoridae family
   j. Paratylenchidae family (pin nematodes, including the genus *Paratylenchus*)
   k. Trichodoridae family (stubby-root nematodes, including the genus *Trichodorus*)
   l. Longidoridae family;
5. Epidermal and root hair feeders
   m. Tylenchidae family (bulb and stem nematodes, including *Ditylenchus dipsaci*)
   n. Atylenchidae family.
The cyst and root knot nematodes are of most economic importance.

Cyst nematodes are nematodes of the Heteroderidae family. They represent an important group of pest organisms in agriculture. Subfamilies are the Heteroderinae, Meloidoderinae (one genus, one species) and Ataloderinae (4 genera, 12 species). The Heteroderinae are divided into 85 species in 7 genera, three of which comprise economically important species: *Heterodera*, 56 species, including *H. schachtii* (sugar beet), *H. avenae* (oat), *H. bifenestra* (grass), *H. cruciferae* (cabbage), *H. glycines* (soybean), *H. goettingiana* (pea), *H. oryzae* (rice), *H. sacchari* (sugarcane), *H. trifolii* (clover, sugar beet) and *H. zeae* (maize); *Globodera*, 13 species, including *G. rostochiensis* and *G. pallida* (potato), *G. solanacearum* and *G. tabacum* (tobacco); and *Punctodera*, 3 species, including *P. punctata* (grass).

Root-knot nematodes (Meloidogynidae) include the economically important genus *Meloidogyne. Meloidogyne* species are polyphagous. They produce conspicuous knots or gall-like swellings on roots. They attack most agriculturally important plants such as vegetables, cotton, strawberries, orchard trees, garden and ornamental plants. Losses can be severe, and can be several generations (the number depending on conditions) per year. Species in temperate zones comprise *M. hapla* (northern root-knot nematode), *M. chitwoodi* and *M. naasi*. In (sub)tropical zones *M. incognita, M. javanica* and *M. arenaria* occur.

Sedentary plant parasitic nematodes feed from their host by transforming root cells into multinucleate feeding cells. In the case of cyst nematodes this feeding site consists of a large syncytium, which results from a fusion of adjacent root cells. The successful formation and exploitation of these feeding cells involves a complex interaction between nematode and host plant, in which nematode secretions of both polypeptide and polysaccharide nature are considered to play an important role.

Feeding cells of root-knot nematodes are so-called giant cells. Juveniles of *Meloidogyne* migrate intercellularly in the root to the region of cell differentiation and locate cells in the vascular cylinder of the stele. The juveniles inject stylet secretions into five to seven cells to transform these cells into specialised feeding sites called giant-cells. The multinucleate giant cells become the permanent feeding site for the parasite throughout its life-cycle. Giant cell formation is one of the most complex responses elicited in plant tissue by any parasite. Cells parasitised by juveniles undergo repeated division of their nuclei without cell division. Each host cell fed upon increases dramatically in size, the central vacuole diminishes in size, the cytoplasm increases in volume and density, and cell wall ingrowths form, giving the cells a phenotype similar to that of transfer cells. Giant cells are induced and maintained in susceptible hosts only by the feeding activities of *Meloidogyne* species. Stylet secretions of root-knot nematodes regulate specific host genes affecting protein synthesis, nuclear division, cell growth and differentiation, and cell wall synthesis.

Most plant parasitic nematodes have three large esophageal gland cells, one dorsal and two subventral. They inject secretions from these esophageal (also referred to herein as salivary) glands via their stylet into the cells of their host plant. In the case of sedentary nematodes, these saliva proteins are presumably involved in feeding site induction and they are also necessary for feeding itself, as follows from the formation of intracellular feeding tubes.

### Summary of the invention

The invention is based on the discovery of cellulolytic enzymes produced by nematodes in their salivary glands. These enzymes play a key role in disrupting cell walls when produced by micro-organisms. They probably play a similar role in root parasitism by endoparasitic nematodes. In addition, these enzymes may be multifunctional and may therefore play a role in the induction of feeding cells which are formed by species belonging to the plant parasitic nematodes. Thus, the invention pertains to cellulotytic peptides and parts thereof and their use, as well as to nucleotides encoding them

Previously, cellulase activity was detected in the migratory endoparasite *Bursaphelenchus xylophilus* (see Odani et al. 1985; Kojima et al. 1994; and Deubert et al. 1971. No sequence information of plant nematodal cellulase-type enzymes has been available thus far.

### Detailed description of the invention

The invention pertains to novel peptides having cellulase (endo-β-1,4-glucanase) activity and exhibiting at least 40% amino acid identity in the primary structure with the amino acid sequence of the cellulolytic subventral esophageal proteins (*svp*'s) of *Heterodera glycines* and/or *Globodera rostochiensis*, which are shown in SEQ ID NO.'s 1, 2, 3 and 4. By amino acid homology is meant here amino acid identity in the primary structure. Amino acid similarity is usually higher than the figures given for identity.

In another aspect the peptides are characterised by comprising a series of at least 7 contiguous amino acids of the amino acid sequence shown in one of SEQ ID NO.'s 1, 2, 3 and 4. In particular, said amino acid identity in the primary structure is at least 50%, especially at least 60%, or said series comprises at least 8, especially at least 10 or even at least 12 contiguous amino acids of the amino acid sequence shown in one of SEQ ID NO.'s 1, 2, 3 and 4. The peptide according to the invention preferably corresponds to a nematodal peptide or part thereof, especially to a peptide of a sedentary nematode. The cyst and root-knot nematodes are most important and thus their cellulolytic enzymes are a preferred group according to the present invention. Parts of the peptides especially refer to immunogenic parts, capable of inducing antibodies against the peptides.

The invention furthermore relates to a nucleotide sequence encoding a peptide having cellulase activity and exhibiting at least 40% amino acid homology with the amino acid sequence shown in one of SEQ ID NO's 1, 2, 3 and 4 or hybridising under stringent conditions with a nucleotide sequence shown in SEQ ID NO's 1, 2, 3 and 4, or a part thereof having at least 15, preferably at least 21, more preferably at least 24 or even at least 30 nucleotides encoding an amino acid sequence shown in SEQ ID NO's 1, 2, 3 and 4.

In this context, stringent hybridisation conditions are as follows: hybridisation in 6 x SSC (20xSSC per 1000 ml : 175.3 g NaCl, 107.1 g sodium citrate.5H₂O, pH 7.0), 0.1% SDS, 0.05% sodium pyrophosphate, 5* Denhardt's solution (100 x Denhardt's solution per 500 ml : 10 g Ficoll-400, 10 g polyvinylpyrrolidone, 10 g Bovine Serum Albumin (Pentax Fraction V)) and 20 µg/ml denatured herring sperm DNA at 65°C for 18-24 hrs followed by one 2xSSC, 0.5% SDS (5 min.) wash and one 2xSSC, 0.1% SDS wash at room temperature, one 0.1xSSC, 0.5% SDS at 37°C and a final wash with 0.1xSSC, 0.5% SDS at 65°C.

The coding sequences may contain mutations (insertions, deletions or both) which serve to modify the structure and/or the activity of the expression product. For an active expression product, the minimum identity and/or the hybridisation characteristic as defined above should preferably be maintained. The nucleotide sequence may also correspond to regulating or signal sequences of nematodal cellulases. The nucleotide sequence may comprise substantially the encoding and/or regulating sequences of the cellulase. On the other hand, the nucleotide sequence may be used as a primer or probe in detecting nematodal cellulase encoding sequences.

Also part of the invention are expression vectors and plasmids containing the nucleotide sequences described above under the control of a homologous or heterologous promoter.

Furthermore, the invention is concerned with the use of these sequences for the production of cellulases by a different host under the control of its own, or heterologous regulatory sequences. The expression vectors and host cells may contain multiple copies of the cellulase-encoding sequences (altered or not with respect to SEQ ID NO's 1, 2 or 3) and also of other genes.

Host organisms may be homologous production strains or alternatively heterologous hosts. Suitable host organisms include fungi, yeasts, bacteria and plants. Examples are *Aspergillus* species, *Trichoderma* species, *Bacillus* species, *Kluyveromyces* species and *Saccharomyces* species. For food applications of the cellulase, the host organism is preferably food-grade.

Examples of own control regions and heterologous regulatory regions include fungal constitutive and/or inducible promoters such as the pyruvate kinase promoter (pkiA) and the glyceraldehyde-3-phosphate dehydrogenase (gpd) promoters. Examples of strong yeast promoters are alcohol dehydrogenase, 3-phosphoglycerate kinase and triose phosphate isomerase promoters. Examples of bacterial promoters are α-amylase, *spo2* and promoters of extracellular protease genes.

The host cell may advantageously express or overexpress other relevant proteins, including enzymes, in particular other glycanolytic enzymes such as xylanases, amylases and glucanases, and/or other enzymes such as oxidoreductases such as hexose oxidase, α-glucuronidase, lipases, esterases, and/or proteases. The corresponding genes may be under the control of homologous control regions or under the control region of the cellulase gene.

The invention is also concerned with antibodies directed at the cellulolytic enzymes described above or to immunogenic parts thereof. These antibodies or one or more of the variable parts thereof can be used in protecting crops against the action of parasitic plant nematodes, especially sedentary nematodes, either as such or as an antibody-encoding insert in a transgenic plant.

SEQ ID NO's 1 and 2 an *svp*-encoding gene of 1 kb and 1.5 kb, respectively, of *Heterodera glycines*, with deduced amino acid sequence; these listings show the complete protein, but for a few amino acids at the N-terminus. SEQ ID NO's 3 and 4 depict the N-terminal part of the amino acid sequence of the gene products of *svp*39 and *svp*32, respectively, of *Globodera rostochiensis*. The genes show a high degree of homology. Both genes from *H. glycines* show a homology of 80% on amino acid level. The H.gly 1.5kb gene encodes a protein showing a remarkable glycine/serine rich region from amino acids 302 to 352. This protein can therefore be divided in three regions: amino acids 1-301, 302-352 and 353-485. The gene G.ros39 has a homology at amino acid level (for the part sequenced) of 71% and 79% with the genes H.gly 1kb and H.gly 1.5kb respectively.

The first region of the H.gly 1.5kb gene having a homology of 80% with the protein encoded by H.gly 1kb, shows homologies with the following proteins:
Endoglucanase Z precursor (endo-1,4-β-glucanase; cellulase) from *Erwinia chrysanthemi*; 36% in 361 amino acids overlap (AC P07103)
Endoglucanase precursor (endo-1,4-β-glucanase; cellulase) from *Clostridium acetobutylicum*; 38.3% in 363 amino acids overlap (AC P15704)
Endoglucanase precursor (endo-1,4-β-glucanase; alkaline cellulase) from *Bacillus* sp. (Strain 1139); 34.5% in 171 amino acids overlap (AC P06564)
Endoglucanase precursor (endo-1,4-β-glucanase; cellulase) from *Bacillus subtilis*; 35.2% in 364 amino acids overlap (AC P07983)
Endoglucanase E-5 precursor (endo-1,4-β-glucanase E-4; cellulase E-5) from *Thermomonospora fusca*; 34.4% in 280 amino acids overlap (AC Q01786)
Endoglucanase precursor (endo-1,4-β-glucanase; carboxymethyl-cellulase; CMCase; cellulase) from *Bacillus subtilis*; 35.7% in 364 amino acids overlap (AC P10475)
Endoglucanase IV (endo-1,4-β-glucanase; cellulase; EG-IV) from *Ruminococcus albus*; 33.0% in 297 amino acids overlap (ACQ07940)
Endoglucanase precursor (endo-1,4-β-glucanase; carboxymethyl-cellulase; CMCase; cellulase) from *Bacillus subtilis*; 35.4% in 364 amino acids overlap (AC P23549)
Endoglucanase CELA precursor (endo-1,4-β-glucanase; cellulase) from *Streptomyces lividans*; 35.4% in 297 amino acids overlap (AC P27035)
Endoglucanase A precursor (endo-1,4-β-glucanase; cellulase; EGA) from *Butyrivibrio fibrisolvens*; 36.5% in 277 amino acids overlap (AC P22541)
Endoglucanase A (endo-1,4-β-glucanase; cellulase; clone PNK1) from *Bacillus* sp. (Strain N-4) 37.2% in 328 amino acids overlap (AC P06566)
Endoglucanase B (endo-1,4-β-glucanase; cellulase; clone PNK2) from *Bacillus* sp. (Strain N-4) 35.8% in 332 amino acids overlap (AC P06565)
Endoglucanase C precursor (endo-1,4-β-glucanase; cellulase) from *Bacillus* sp. (Strain N-4) 31.6% in 187 amino acids overlap (AC P19570)
Endoglucanase precursor (endo-1,4-β-glucanase; cellulase; alkaline cellulase) from *Bacillus* sp. (Strain KSM-635) 28.7% in 188 amino acids overlap (AC P19424)

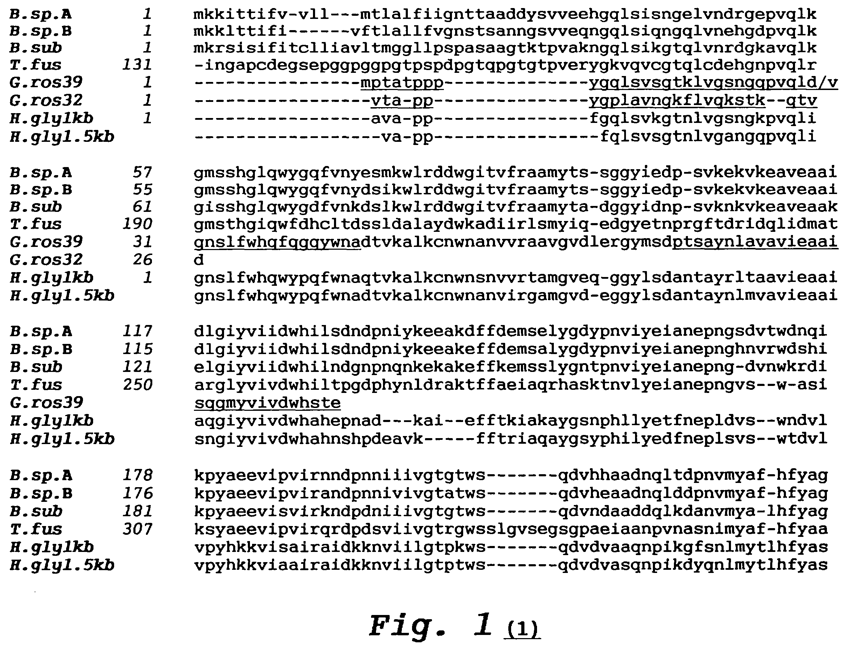

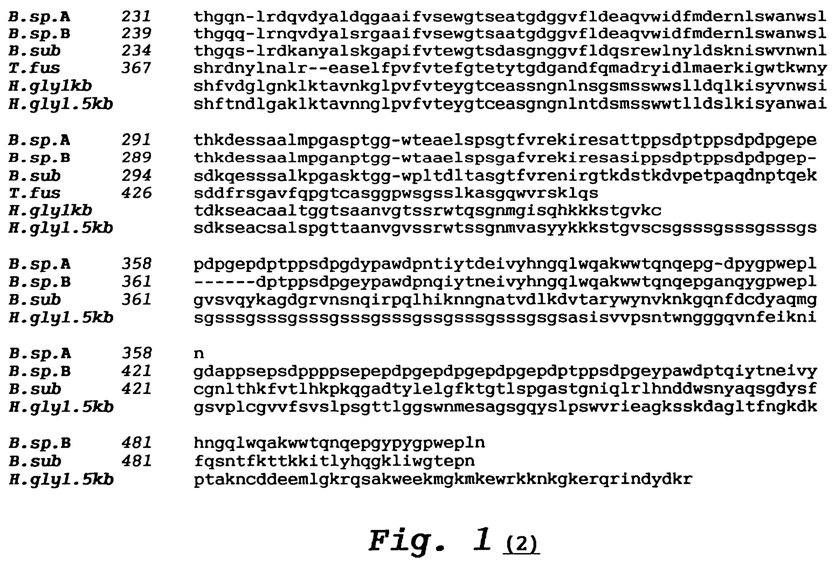

From the described homologies it is concluded that the present genes encode a endo-1,4-β-glucanase (EC 3.2.1.4) or cellulase. Figure 1 shows an alignment with three known cellulases, two from the alkalophilic *Bacillus* strain N-4 (AC P06565, AC P06566) and one from *Thermomonospora fusca* (AC Q01786). Two sites have been indicated: proton donor and nucleophile. The enzymes may show multifunctionality including e.g. cellobiohydrolase, lichenase, xyloglucanase or xylanase activity.

The cellulase according to the invention can be used in enzyme preparations.The invention is also concerned with such enzyme preparations. Such preparation may further contain other enzymes, such as other glucanases, xylanases, amylases, lipases, proteinases, oxidoreductases etc.

Cellulase containing enzyme preparations can be applied:
- in treating cellulosic fibres to improve colour and feeling and various other properties, while preventing a lowering in strength of the cellulosic fibres ( 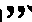 loss in weight treatment 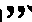 or 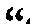 stone-free stone-washed process 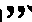 ), as described in WO 94/19528);
- as additive in detergents for clothing, acting only on the non-crystalline region to remove soils and not having an effect of improving colour or feeling of the cellulosic fibre (see also WO 94/19528);
- as additive to feed for domestic animals to more effectively utilise the nutritive components, thus improving feed growth and feed conversion, as described in EP-A-674843;
- for increasing the filtration rate of beer by degrading cellulosic fibres;
- for clarification of fruit juices and in vegetable processing;
- as a bread improver and in bakery applications;
- as a modifier for cellulose for use in dietary food, clinical foods and medicaments;
- for waste water treatment in the pulp and paper industry and in the starch industry; the cellulase assists in removing cellulose residues and also facilitates drainage in paper making and deinking of paper (see WO 94/00578 and WO 96/19569).

### Examples

### General: Sequencing of NH₂-termini of svps

### Sample preparation

1-3.10⁶ J₂ of *G. rostochiensis* or *H. glycines* were homogenised in Potter Elvejhem homogeniser and teflon pestle (750 rpm; 40 times up-and-down) in 10 mM Tris-Cl pH 7.4. Pefabloc (AEBSF, Boehringer Mannheim) was added to a final concentration of 1 mM. The homogenate was centrifuged for 5 min. at 14.000 rpm (16.000 x g), and the supernatant and the pellet underneath the fat layer were collected. Shortly before use 1/3 sample volume of 4x SDS-sample buffer (240 mM Tris-Cl (pH 6.8), 40% glycerol, 8% SDS, 0.1 % bromophenol blue, 20 % 2-mercaptoethanol) was added, the sample was heated for 3 min at 100 °C and centrifuged for 5 min. at 14.000 rpm (16.000 x g). The overlaying supernatant was used in the preparative purification.

### Purification of proteins

A 10% SDS-polyacrylamide gel was poured in a preparative electrophoresis system (Prep Cell, Bio-Rad, Richmond, Ca, USA) and overlaid with a 4% stacking gel according to the manufacturer. The 28 mm diameter gel tube was used. Standard SDS-PAGE buffers were used (Laemmli, 1971). The following running conditions were applied: 40 mA constant current (about 280 V). After 2:00 h elution was started at 1 ml/min and collected in a 50 ml tube. After approximately 2:35 h the bromophenol blue migrated into the elution chamber, whereafter the fraction collector was started and 2 ml per fraction was collected. After about 6:00 h and 100 fractions collected, electrophoresis was stopped. 25 µl of each third fractions was kept separate for a SDS-PAGE and immunoblot, the remaining part stored in the -80 °C freezer. After analysis on a SDS-PAGE slab gel ann immunoblot (see de Boer et al. 1996) the fractions containing the *svp*s were combined to three pools of 31/32 kDa, 39 kDa and 49 kDa.

An immunoaffinity column was prepared of monoclonal antibody MGR 48 (de Boer et al., 1996) by cross-linking it to protein A Sepharose® 4 Fast Flow (Pharmacia, Uppsala, Sweden) using dimethyl pimelimidate (Sigma, St. Louis, MO, USA). The pooled *svp* samples were dialysed against 20 mM Tris-HCl pH 8 overnight (two 2 litre buffer changes) and, one sample at a time, loaded onto a, with the same buffer, equilibrated MGR48 protein A affinity column, at 1 ml/min. The sample was 'reloaded 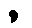 until the UV (280 nm) signal remained constant. The column was then equilibrated with 20 mM Tris-HCl pH 8 to the base line whereafter 3 column volumes of 0.2 M glycine pH 3 were applied for elution. The UV 280 nm peak observed was collected and the pH immediately adjusted to pH = 7 with 1.0 M Tris-HCl pH 8. The column was regenerated with 20 mM Tris-HCl pH 8 whereafter the two other samples of svp 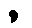 s were subsequently purified.

### Sample preparation for NH₂-terminal sequencing

To the protein samples Pefabloc was added to a concentration of 1 mM whereafter they were concentrated by lyophilisation. The concentrated samples were subjected to SDS-polyacrylamide gel electrophoresis using a Tris/tricine buffer system (Schagger, H. and von Jagow, G. 1987) whereafter the proteins were transferred to an PVDF-membrane (Immobilon-Millipore, Bedford, MA) by semi-dry blotting. The blot was stained with Coomassie Brilliant Blue R250, stained bands were cut out and destained with 90% n-propanol.

### Example 1: Cloning of svps from H. glycines

From 1.10⁶ water hatched second stage juveniles of *H. glycines* RNA was isolated using the RNA Extraction Kit (Pharmacia Biotech, Uppsala, Sweden). Subsequently, cDNA was synthesised and the target DNA amplified using the 3 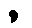 RACE System for Rapid amplification of cDNA Ends (Gibco-BRL, Life Technologies, Gaithersburg, MD, USA). For amplification of the putative cellulase gene the following degenerate primer was used:
5 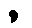 -GTIGGIGCIAA(C/T)AA(A/G)CA(A/G)CCIGTICA-3 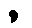

PCR resulted in two bands of approximately 1000 and 1500 basepairs respectively. Both bands were purified from the agarose gel using Easy prep PCR product prep kit (Pharmacia, SE) and ligated in the TA cloning vector (Invitrogen, San Diego, CA, USA). DNA sequencing (ALF automated sequencing, Pharmacia Biotech, SE) using Thermo Sequenase fluorescent labelled primer cycle sequencing kit with 7-deaza-dGTP (Amersham, Buckinghamshire, UK) resulted in the sequences according to SEQ ID No's 1 and 2. The sequences show a homology with cellulase from other organisms as described above.

**Example 2: Cloning of svp** 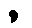 **s from *G. rostochiensis***

From 1*10⁷ juveniles of *G. rostochiensis* hatched in potato root diffusate RNA was isolated using RNA Extraction Kit (Pharmacia Biotech, SE). PolyA RNA was purified using mRNA Purification Kit (Pharmacia Biotech, SE). A cDNA library containing 2.5*10⁶ primary recombinants with an average insert size of 1500 basepairs was constructed by Invitrogen (San Diego, CA, USA). This library in cloning vector pcDNAII was used as target DNA for amplification of svp encoding sequences using primers:
5 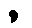 -CCATACGGGCAATTGTCCGTCTCCGGCACC-3 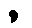 vs.
5 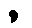 -AAAGGGGGATGTGCTGCAAGGCG-3 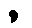 , and
5 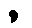 -TGCCA(A/G)TCIAC(G/A/T)AT(A/G/C/T)AC(A/G)TACT(G/T)CC-3 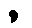 vs.
5 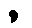 -GCTTCCGGCTCGTATGTTGTGTG-3 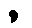 .

PCR resulted in two bands approx. 650 and 750 bp respectively. Both bands were purified from agarose using Easy prep PCR product prep kit (Pharmacia, SE) and ligated in a TA cloning vector of the One shot cloning kit (Invitrogen, San Diego, USA). DNA sequencing resulted in sequences according to SEQ ID No's 3 and 4.

### Example 3: Presence of cellulase in Meloidogyne species

Genomic Southern blots of *M. incognita*, *M. arenaria*, *M. javanica*, *M. hapla*, *H. glycines*, *Caenorhabditis elegans* (reference nematode) and tomato were prepared and hybridised with a probe containing the nucleotides 350-1079 of SEQ ID NO 1, as described by Ray et al, 1994. A single band appeared in the Southern blot of *M. incognita*, *M. arenaria* and *M. javanica* indicating the presence of cellulase in these nematode species. Similarly, on a Northern blot of poly (A)+ RNA of *M. incognita* (Ray et al, 1994) a single band was observed with a molecular weight between 2000 and 3000 basepairs.

### Example 4: Functionality assay I

Carboxymethylcellulose (CMC), 0.4% in 50 mM K-phosphate/citric acid buffer pH 5.2 (PCA buffer) was mixed with an equal volume of 1% agarose (Seakem) in PCA buffer and a layer of 4 mm was poured in a 10 cm petri dish (Mateos et al. 1992). Holes were punched out able to contain 4 µl. From a homogenate of second stage larvae of *G. rostochiensis* in 50 mM Na-phosphate buffer pH 7.2 containing 16 µg/µl protein, 3 µl was added to a hole. After 18 h incubation at 30 °C the plates were flooded with 0.1% Congo Red in water and incubated for 30 min. at room temperature and then rinsed with 1 M NaCl. A translucent halo with a diameter of 16 mm was observed proving the presence of cellulolytic activity in nematode homogenates. This sample could be 256-fold diluted before the activity had become invisible.

When the same experiment was carried out with 20-fold concentrated salivary gland secretions (potato root diffusate wherein second stage juveniles were hatched), also cellulolytic activity was observed. A translucent halo with a diameter of 8 mm was seen.

### Example 5: Functionality assay II

To establish whether the cellulase activity in potato cyst nematode homogenate and in collected salivary secretions correlate with the antigens detected by the monoclonal antibody MGR48 and thus with the gene cloned as described in example 3, both samples were subjected to electrophoresis on a 10% polyacrylamide gel containing 20 µg per ml bovine serum albumin. Samples were prepared with a sample buffer having a final SDS concentration of 0.4% while a reducing agent was omitted. Of the potato cyst nematode homogenate 24 µg of protein and 15 µl of 20-fold concentrated salivary gland secretions (potato root diffusate wherein second stage juveniles have been hatched) were loaded in duplicate without having been boiled. After the run half of the gel was blotted onto PVDF membrane and immunostained using MGR48. The other half was washed 3 times in 100 ml PCA buffer for 40 minutes and then placed on top of a 1 mm layer of 0.5 % Seakem agarose containing 0.2% carboxymethylcellulose in PCA buffer. After overnight incubation at room temperature the gel was removed and the agarose layer stained with Congo Red for 30 minutes. After destaining by rinsing with 1M NaCl clear areas representing cellulase activity became visible. The position of these translucent halos for the protein extract corresponded to that of the 49 and 32/31 kD bands detected on western blot with MGR48. In spit two halos became visible and their positions were the same as the 32/31 kD and the 39 kD bands on the immunoblot. This result demonstrated that the cDNA molecules cloned from the PCN cDNA library indeed encode proteins with cellulase activity.

### References

De Boer, J.M., G. Smant, A. Goverse, E.L. Davis, H.A. Overmars, H. Pomp, M. Van Gent-Pelzer, J.F. Zilverentant, J.P.W.G. Stokkermans, R.S. Hussey, F.J. Gommers, J. Bakker, and A. Schots, Secretory granule proteins from the subventral esophageal glands of the potato cyst-nematode identified by monoclonal antibodies to a protein fraction from the second-stage juveniles (1996) *Molecular Plant-Microbe Interactions* **9**, 39-46.
Deubert, K.H., & R.A. Rohde. 1971. Nematode enzymes (Chapter 16). *In:* Plant Parasitic Nematodes. Volume II. Eds. Zuckerman, B.M., W.F. Mai, & R.A. Rohde. Academic Press. pp. 73-90.
Kojima K., Kamijyo A., Masumori M., & Sasaki S. 1994. Cellulase activities of pine-wood nematode isolates with different virulences. *Journal of the Japanese Forestry Society* **76** (3): 258-262.
Mateos, P.F., Jimenez-Zurdo, J.I., Chen, J., Squartini, A.S., K¦Haack, S.K., Martinez-Molina, E., Hubbell, D.H. and Dazzo, F.B. 1992. Cell-associated pectinolytic and cellulolytic enzymes in *Rhizobium leguminosarum* bivar *trifolii*. *Appl. Environ. Microbiol*. **58**, 1816-1822.
Odani, K., Sasaki, S., Nishiyama Y. & Yamamoto N. 1985; Early symptom development of the pine wilt disease by hydrolytic enzymes produced by the pine wood nematodes - cellulase as a possible candidate of the pathogen. *Journal of the Japanese Forestry Society* **67** (9): 366-372.
Ray, C., Abbott, A.G., and Hussey, R.S. 1994 *Mol. Biochem. Parasitol*. **68**, 93-101.
Schagger, H. and von Jagow, G. 1987. Tricine-sodium dodecyl sulfate-polyacrylamide gel electrophoresis for the separation of proteins in the range from 1 to 100 kDa. *Anal. Biochem.* **166**, 368-379.

It has been found now that cyst nematodes can be effectively controlled by means of antibodies which are directed against the (salivary) esophageal proteins of the nematode, especially against the subventral esophageal proteins (*svp*'s). Thus, the invention is primarily related to antibodies directed against a salivary protein of the nematode or at an immunogenic part or equivalent thereof. According to the present invention, "directed against" means that the antibody interacts with the particular protein; in particular it means that the antibody inhibits the functioning of said protein.

Salivary proteins of cyst nematodes that are most suitable as a target of antibody inhibition have an apparent molecular weight of about 28-52, more in particular 30-50 kDa. These proteins are produced by second stage juveniles (12) of the cyst nematode and occur especially in the subventral salivary glands of the nematode. Individual proteins have molecular weights of about 30 kDa (*svp*30), 31 kDa, (*svp*31a), 31 kDa (*svp*31b), 32 kDa (*svp*32), 39 kDa (*svp*39), and 49 kDa (*svp*49), in case of *G. rostochiensis* salivary proteins, with corresponding isoelectric points (pI) of 7.6, 7.6, 7.9, 6.8, 7.3 and 8.7, respectively. In other species, such as *H. glycines*, these chararcteristics are similar. These proteins are related, probably in that the lower molecular weight proteins are processed forms of the 49 kDa protein, and they share at least one epitope. At least one of the *svp*31 appears to be glycosylated, and it is assumed that they are glycosylated variants of *svp*30. The other four proteins are probably not glycosylated, since they are not affected by enzymic de-glycosylation.

The antibodies according to the invention are preferably directed against proteins derived from second-stage juveniles of the particular nematode. Preferably, the antibody reacts with more than one of the six proteins referred to above, in that it has an epitope shared by two or more up to six of the proteins. A very useful antibody was found to be the antibody denoted herein as MGR 48, which recognises the six proteins *svp*30, *svp*31a, *svp*31b, *svp*32, *svp*39 and *svp*49, as confirmed by Western blotting.

The antibodies can also be directed at other parts of the proteins defined above. They are preferably directed at a continuous polypeptide epitope, not at a carbohydrate moiety, nor at a disulphide moiety.

The antibody to be used according to the invention may be complete, multimeric antibodies, i.e. antibodies which contain all four subunits. The antibody may also be partial antibodies which comprise one or more variable parts of the complete antibody. In particular, the antibody may comprise the variable part of the light chain (V_{L}) and the variable part of the heavy chain (V_{H}), preferably fused by a linker peptide. Such a single chain antibody (scFv) also requires one gene for expression, whereas a complete antibody requires at least two genes.

The antibodies according to the invention may be monovalent, i.e. contain one antigen-binding site, or bivalent (two binding sites). Bivalent antibodies may be obtained e.g. by coupling two single-chain (scFv) antibodies by means of a linker, or by shortening the linker peptide between the variable parts of an scFv so as to induce dimerisation of scFv's, or by providing scFv's with a tail sequence leading to oligomerisation of antibody chains. They may be monospecific or bispecific, i.e. contain variable parts reacting with a single antigen or with two different antigens.

The amino acid sequence of two variable regions of an antibody specifically reacting with a salivary protein of cyst nematodes, fused by a 15-mer linker peptide, as well as the nucleotide sequence encoding this amino acid sequence are depicted in SEQ ID No. 5. Instead of the Gly-Ser linker peptide shown in SEQ ID No. 5 (amino acids 113-127), another linker such as the 16-mer linker 202, depicted in SEQ ID No. 6, may be used. The amino acid and nucleotide sequences of a signal peptide that can be used for excretion of an antibody according to the invention is depicted in SEQ ID No. 7.

The invention also relates to polypeptides corresponding to the salivary proteins as mentioned above or to an immunogenic part thereof having at least 5, preferably at least 8 amino acids, as well as to nucleotide sequences of at least 15, preferably at least 24 nucleotides, encoding such a polypeptide. Such polypeptides can comprise an amino acid sequence having a degree of homology of at least 50%, preferably at least 60%, more preferably at least 75% with the amino acid sequence depicted in SEQ ID No's 1 to 4.

The invention also relates to peptides corresponding to the antibodies described above or to parts thereof, especially to variable domains thereof, such as depicted in SEQ ID No. 5 and equivalents and parts thereof. Suitable parts include the complementarity determining regions (CDR) corresponding to amino acids 25-39, 55-61 and 94-102 (light chain), ad 158-162, 177-193 and 226-232 (heavy chain), and combinations thereof. Also covered by the invention are nucleotide sequences encoding such peptides, such as given in SEQ ID No. 5. Variable domains are e.g. comprised by the partial nucleotide sequences 1-336 and 382-819 of SEQ ID No. 5.

The invention is further concerned with expression systems comprising at least such an antibody-encoding nucleotide sequence and a sequence regulating expression of said nucleotide sequence. These expression systems may be constructed by first producing antibodies against a salivary protein or immunogenic part thereof, followed by isolating the antibody and determining the nucleotide sequence of the gene encoding the antibody peptide, and then synthesising a polynucleotide corresponding to that antibody gene or part thereof and combining it with one or more regulatory sequences. The expression system according to the invention may also comprise other genes, which other genes may encode other peptides having a plant protecting or regulating function.

Also comprised by the invention is a process for protecting a plant against the action of a cyst nematode, wherein the plant is transformed using a nucleotide sequence encoding an antibody or part thereof or an expression system as described above. Furthermore, transgenic plants containing in their genome such a nucleotide sequence and capable of expressing said sequence are part of the invention. Such transgenic plants are resistant to cyst nematode attack. Antibodies against a salivary protein of a cyst nematode, when expressed in plants, are also part of the invention.

Gene constructs to be used may comprise nucleotide sequences encoding the complete antibody molecule, the Fab part, scFv part or any other part (like complementarity determining regions) which shows binding to the target proteins.

The desired cellular location of the proteins can be achieved using the appropriate signal sequences. The signal sequence is responsible for targeting the protein from the cytoplasm to aother subcellular compartment. An example of a targeting route is the secretion via endoplasmic reticulum and golgi apparatus. Examples of signal sequences for secretion are described in Briggs and Gierasch (1986), Firek et al. (1993), Düring et al. (1990) and Shirasu et al. (1988). SEQ NO. 7 shows the nucleotide and amino acid sequences of a suitable signal peptide; the particular signal peptide shown is that of the kappa light chain of antibody CEA 66E3'CL (see Kabat et al. 1991 and Cabilly et al. 1984) with Glu at position 23 substituted by Asp.

The antibody-encoding genes can be expressed in plants under the control of any type of promoter which is active in plants. Examples are:
a) constitutive promoters such as the CaMV-35S (Kay et al. 1987);
b) tissue specific promoters such as described by Nap et al. (1993) (leave), De Almeida et al. (1989) (leave, SSU-promoter), Nap et al. (1992) (potato tuber, patatin promoter), Hendriks et al. (1991) (potato tuber), Guerche et al. (1990) (seed);
c) inducible promoters such as the TR2' promoter (Langridge et al. 1994), wound inducible promoters (Logemann et al. 1989; Suh et al. 1991) or chemically induced promoters (Williams et al. 1992).
   Promoters may be used singly, in tandem or as divergent promoters.

Transformation can be done using any method which ensures a stable integration of the antibody encoding gene in the plant genome in such a way that it can still be transcribed. Examples of transformation methods are:
a) *Agrobacterium tumefaciens* mediated transformation (Horsch et al. 1985): based on a natural transformation system in which the bacterium stably incorporates part of a plasmid DNA (T-DNA) into the plant genome. The T-DNA includes the gene to be expressed.
b) Microprojectile bombardment (Vasil et al. 1992): particles coated with DNA penetrate the plant cell nuclei at high velocity where the DNA is integrated into the genome by host recombination processes.
c) Tissue electroporation (D'Halluin et al. 1992): under the influence of a strong electric field DNA penetrates the plant cells and, after being transported to the nuclei, it is incorporated into the plant genome by host recombination processes.

### Further examples

### Materials and methods

Nematodes: Second stage juveniles (J2) of *Globodera rostochiensis* pathotype Ro1, and of *G. pallida* pathotype Pa2 were hatched for 7 days by soaking cysts on a 100 µm sieve in potato root diffusate (Clarke & Perry, 1977). The J2 suspensions were mixed with an equal volume of 70% (w/v) sucrose in a centrifuge tube. The 35% sucrose mixture was covered with a layer of demineralised water, and centrifuged briefly at 1000 g. Purified juveniles were collected from the sucrose-water interface with a pasteur pipette, washed with tap water and used for further processing in the various experiments.
Preparative electrophoresis: In total 2.75 million J2 of *G. rostochiensis* were homogenised in 208 mM Tris-HCl pH 6.8 supplemented with 8.33% (v/v) 2-mercaptoethanol (ME) at 5 °C using a small glass mortar and pestle. The J2 were homogenised in aliquots of approximately 300,000 individuals each. Following homogenisation the samples were pooled and mixed in a ratio of 3:2 (v:v) with a solution of 5% SDS / 25% glycerol / 0.1% Bromophenol Blue, thus producing standard SDS-sample buffer (O'Farrell 1975) The homogenate was heated for 5 minutes in boiling water, centrifuged for 5 min. at 10000 g, and the supernatant was stored at -80 °C until use. The nematode sample (2 ml, approximately 13 mg of protein) was fractionated by preparative SDS-PAGE using a Model 491 Prep Cell apparatus (Bio-Rad, Richmond, California, USA), essentially according to the instruction manual, with the correction that 0.1% SDS was added to the buffers of the acrylamide gels. The cylindrical separating gel was 50 x 28 mm (height x diameter) and contained 10% acrylamide. The stacking gel contained 4% acrylamide and was 15 mm in height. The proteins were separated with a current of 40 mA, and 1.5 ml fractions were collected using an elution buffer flow rate of 1 ml/min. The fractions were concentrated by freeze drying, washed three times in phosphate buffered saline pH 7.4 (PBS) using 1.5 ml microcentrifuge filter concentration units with molecular weight cut-off of 5 kDa (Ultrafree-MC, Millipore Corp. Bedford, USA), taken up in 50 µl PBS and stored at -20 °C. The 50 original fractions were pooled in groups of 2 to 5 fractions to form a representative set of 16 samples covering the entire molecular weight range of the fractionation experiment. Each pooled sample was diluted in PBS to obtain three 200 µl aliquots for successive immunisations.
Immunisations and cell fusions: Sixteen mice were immunised intraperitoneally with the isolated protein fractions, which were mixed 1:1 with Freund's incomplete adjuvant. After four weeks a second immunisation was given, also with incomplete adjuvant. Two weeks later antiserum samples were collected for immunofluorescence microscopy. One mouse that was selected for monoclonal antibody production received a final booster injection 12 weeks after the first immunisation. Three days later the mouse was sacrificed, and MAb-producing hybridoma cell lines were obtained by fusing spleen cells with SP 2/0 myeloma cells (Goding 1983; Schots et al. 1992b).
Immunofluorescence microscopy: Preparation of J2 for indirect immunofluorescence testing of MAbs and antisera was essentially according to Atkinson et al. (1988) and Hussey (1989). J2 of *G. rostochiensis* were fixed in 2% paraformaldehyde in PBS pH 7.4 for 3 days. The nematodes were then washed in distilled water, and drops of concentrated suspension were spread evenly onto aluminium dishes (diameter 2 cm), which were glued to microscope slides for easy manipulation. The drops were allowed to dry at room temperature in a box with silica gel, after which the dishes with nematodes were stored dry at -20 °C. For screening of the MAbs, the dried J2 were cut into small pieces on their aluminium dish using a razor blade. By cutting parallel lines in three different directions, it was assured that most of the nematodes were cut in 2 or more pieces. Then the nematodes were taken up in 1 ml of PBS containing 1 mg/ml proteinase K (Merck, Darmstadt, Germany) in a 1.5 ml micro-centrifuge tube and incubated for 20 minutes with agitation at room temperature. After this, the nematodes were pelleted (2 minutes 2000 g, swing out rotor) and subsequently taken up in cold methanol (1 min; -20 °C) and cold acetone (2 min; -20 °C). After removal of the acetone, the nematode sections were resuspended in blocking buffer containing PBS pH 7.2, 10% horse serum, and 1 mM phenylmethylsulphonyl fluoride. Labelling of the J2 was done in 96 well filtration plates with a pore size of 0.45 µm. (MultiScreen-HV, Millipore Corp. Bedford, U.S.A.). To each well 20 µl of nematode suspension (containing approximately 200 sections) was added, followed by 80 µl of hybridoma culture supernatant or mouse antiserum diluted 1:1000 (v:v) in PBS. After incubation overnight in a moist atmosphere, the nematode sections were washed three times with PBS / 0.1% Tween 20 by applying vacuum to the filtration plates, and they were next incubated for 2 hours with FITC-conjugated rat-anti-mouse IgG (Cat. nr. 415-095-100; Jackson Immuno Research Laboratories Inc, West Grove, USA), diluted 1:100 in PBS containing 0.1% BSA and 0.1% Tween-20. After three washes with PBS / 0.1% Tween-20, the nematode sections were taken up in 20 µl of distilled water, and transferred to 24 well microscope slides (Cat Nr. 10-448-S, Cel-Line Associates Inc., New Field, U.S.A.) precoated with 0.1% poly-L-lysine (2 µl/well). After drying in the dark 2 µl of anti-quenching solution, consisting of 0.5M sodium carbonate buffer pH 8.6 mixed 1:1 with Citifluor Antifadent 2 (Agar Scientific Ltd., Stansted, England), was applied to the wells and the slides were covered with a large coverslip, which was fixed with dots of nail polish. Specimens were viewed with the 50x water immersion objective of a Leitz epifluorescence microscope, using an L 02.1 or I filter block. Hybridoma cell lines producing antibodies to the subventral glands of *G. rostochiensis*, were subcloned to stability, and stored in liquid nitrogen. Isotyping of the light and heavy chains of the MAbs was essentially as described by Schots et al. (1992b). Immunofluorescence testing of J2 from *G. pallida* and *M. hapla* followed the same procedure as described for *G. rostochiensis*, with the exception that the initial fixations in paraformaldehyde were different: two days for *G. pallida*, and one day for *M. hapla*. Immunofluorescence testing of J2 from *G. tabacum*, *M. incognita* and *H. glycines*, *H. schachtii* was as described by Davis et al. (1992).
SDS-PAGE and Western blotting: Analytical mini SDS-PAGE was performed essentially as described by De Boer et al. (1992). For the examination of the protein fractions that were obtained with preparative electrophoresis, 4 µl of SDS-sample buffer was added to 2 µl from the concentrated fractions in PBS, and these samples applied to 20 µl slots in the stacking gel. Following electrophoresis, the gels were stained with Coomassie Blue G-250 (Neuhoff et al. 1988). For Western blot testing of MAbs, J2 of *G. rostochiensis* were homogenised as described above, and per minigel approximately 10000 J2 were added to a single 73 mm wide slot in the stacking gel. An adjacent reference well (3 mm wide) was filled with prestained molecular weight markers (Bio-Rad, Richmond, USA). Following electrophoresis (13% separating gel), the proteins were transferred to polyvinylidene difluoride (PVDF) membrane (Immobilon-P, Millipore Corp., Bedford, USA) using a semi-dry blotting apparatus. A continuous transfer buffer system was used containing 39 mM glycine, 48 mM Tris and 20% (v/v) methanol. Transfer was carried out with 0.8 mA/cm² for 1 hour. The blots were cut into strips, which were blocked overnight in PBS pH 7.4 / 0.1% (v/v) Tween-20 (PBST) supplemented with 5% (w/v) defatted milk powder. Following a wash in PBST / 1% milk powder, the strips were incubated for 2 hours in hybridoma culture fluid, diluted 1:6 in PBST / 1% milk powder. For MAb MGR 48 affinity-purified antibody (1 µg/ml) was used instead of culture fluid. After washing three times in PBST / 1% milk powder, the strips were next incubated individually in alkaline phosphatase conjugated rat-anti-mouse IgG (H+L) (Cat. Nr. 415-055-100, Jackson Immuno Research Laboratories Inc, West Grove, USA) diluted 1:5000 in PBST / 1% milk powder for 1 hour. After washing in PBST / 0.1% milk powder (1x) and PBST (3x), the strips were stained individually in 0.1 M ethanolamine-HCl pH 9.6, supplemented with 4 mM MgCl₂, 5-bromo-4-chloro-3-indolyl phosphate (0.06 mg/ml) and nitro blue tetrazolium (0.1 mg/ml).
Electron microscopy: For ultrastructural examination, J2 of *G. rostochiensis* were fixed at 4 °C with 4% paraformaldehyde in 0.1 M HEPES-buffer pH 7.5 for 2 days. The suspension of fixed J2 was spread on a microscope slide and the nematodes were chopped into pieces with a razor blade. The nematode sections were then fixed with subsequently 2% glutaraldehyde and 1% osmium tetraoxide (both in 0.1 M HEPES pH 7.5), and stained with 1% aqueous uranyl acetate. Following stepwise dehydration in ethanol, the J2 were infiltrated for 1 day with Spurr epoxy resin (Spurr, 1969). The nematode fragments were then transferred to fresh epoxy resin in a BEEM capsule, centrifuged to the bottom, and polymerised at 60°C. Thin sections were cut with an LKB ultramicrotome, collected on formvar-coated 100 mesh copper grids, poststained with 2% uranyl acetate in 50% methanol. For immunolabelling, J2 of *G. rostochiensis* were fixed for 2 days at 4°C in 2% paraformaldehyde in PBS pH 7.4. After chopping them into pieces, the J2 were washed 2 times in distilled water, dehydrated in 30%-50%-70%-96% ethanol, infiltrated at room temperature for 1 hour in a 1:1 mixture of 96% ethanol and LR-White acrylic resin (London Resin Co. Ltd., Basingstoke, GB) and subsequently in pure LR-White resin for 4 hours. The nematode fragments were transferred to fresh resin in a gelatin capsule, centrifuged to the bottom, and polymerised at 60 °C. Following ultramicrotomy, thin sections were collected on formvar-coated copper grids and immunolabelled with colloidal gold according to the following protocol (Aurion Immuno Gold Reagents, Wageningen, NL) 10 min. PBS pH 7.6 / 0.05 M glycine, 2 hours affinity purified MAb MGR 48 diluted to 1 µg/ml in incubation buffer (PBS pH 7.6 / 0.2% BSA-C / 20 mM NaN₃), 6x 5 min. wash with incubation buffer, 2 hours colloidal gold solution (10 nm particle size, conjugated with goat-anti-mouse-IgG immunoglobulins; Cat. nr 810.022, Aurion Immuno Gold Reagents, Wageningen, NL) diluted 1:10 in incubation buffer, 6x 5 min. wash with incubation buffer, 3x wash with PBS, and 3x wash with distilled water. The sections were not post-stained. Electron micrographs were taken with a Zeiss EM 109 transmission electron microscope.
Sequencing of variable parts of MAb MGR 48:
   cDNA to mRNA from the hybridoma cell-line producing MGR 48 was produced and cloned into an *E. coli* vector. The single chains were sequenced according to standard methods.
Two-dimensional gel electrophoresis
   Proteins were separated in two-dimensional gel electrophoresis using an immobilised pH gradient and a 8-18% (w/v) acrylamide SDS-PAGE gradient gel (IPG-Dalt), essentially according to the manufacturers' protocol (Immobiline Dry Strips 3-10L, and ExcelGel, Pharmacia LXB Biotechnology, Uppsala, SE). Freshly hatched J₂ of *G. rostochiensis* were homogenised in 1% (v/v) Nonidet P-40 and 2% (v/v) ME. Protein concentration was estimated according to Bradford (1976) using BSA as a standard. Shortly before gel electrophoresis, five volumes of 8 M urea, 0.5% (v/v) Nonidet P-40, 2% (v/v) ME, 2% (v/v) carrier ampholytes pH 3-10 (BioRad), 1 mM Pefabloc (Boehringer Mannheim, D), and a trace of bromophenol blue were added to the sample. This solution was thoroughly mixed, incubated for 1 h at room temperature and centrifuged for 10 min at 14,000 x *g*. The IPG strips (4x110 mm) were rehydrated in 8 M urea, 0.25% (v/v) Nonidet P-40, 10 mM dithiothreitol (DTT), 2 mM acetic acid, and a trace of Orange G for 7 h. The samples (15 µg) were applied 1 cm from the anodic side of the IPG strips. The isoelectric focusing conditions were as follows: 3 h at 300 V, a linear increase from 300 to 2,000 V in 5 h, 8 h at 2,000 V. The temperature was maintained at 14°C. The IPG strips with the focused proteins were equilibrated for 15 min in freshly prepared 10 ml 50 mM Tris-HCl (pH 6.8), 4.17 M urea, 30% (v/v) glycerol, 1% (w/v) SDS, 16.2 mM DTT, and subsequently for 15 min in 10 ml 50 mM Tris-HCl (pH 6.8), 4.17 M urea, 30% (v/v) glycerol, 1% (w/v) SDS, 240 mM iodoacetamide, with a trace of bromophenol blue. Pairs of IPG strips were transferred to a preeast 8-18% (w/v) acrylamide SDS-PAGE gradient gel (ExcelGel 110x245x0.5 mm, Pharmacia) and second dimension electrophoresis was performed at 20 mA for 30 min and at 40 mA for another 75 min. The SDS-PAGE gel was used for semi-dry Western blotting on PVDF membrane at 0.8 mA/cm² for 1 h. Six protein spots were detected on Western blot using MGR48. *Svp*49 focused at pI 8.7, whereas *svp*39 focused at pI 7.3 in the applied pH range. A new protein species (*svp*32) which was not separated on one-dimensional Western blotting before, appeared at pI 6.8 and 32 kDa. *Svp*31 focused as two isoelectric point variants (pI 7.6 and 7.9), while *svp*30 focused at 7.6.

### Results

Antibody production: Fractionation of homogenised J2 of *G. rostochiensis* by preparative continuous flow electrophoresis in the presence of sodium dodecyl sulphate (SDS) yielded 50 protein fractions with average apparent molecular weights ranging from 30 to 52 kDa. Examination of successive fractions by analytical SDS polyacrylamide gel electrophoresis (SDS-PAGE) revealed very narrow protein zones, with a very gradual increase in average molecular weight. Therefore the original fractions were pooled to form a representative set of 16 protein samples of increasing molecular weight, which were used for immunising 16 mice. Antisera collected after the second immunisations with these samples were screened with immunofluorescence microscopy for reaction with the salivary glands of J2 of *G. rostochiensis*. It was found that four mice had produced antisera that reacted with the salivary glands. Because of simultaneous strong staining of additional structures within the J2, it was at this stage not yet possible to determine with certainty whether these antisera labelled the dorsal or the subventral gland. The mouse that was immunised with the 38-40.5 kDa protein sample was chosen for monoclonal antibody production.
Screening of hybridoma's: Screening of the hybridoma culture supernatants by immunofluorescence microscopy resulted in 12 MAbs that bound specifically to the subventral glands of J2 from *G. rostochiensis*. The staining effectiveness of the glands by the antibodies was found to be variable, both between different MAbs in a single experiment, between individual J2 stained with the same antibody, and between repeated experiments. Ten MAbs (MGR 46, 47, 48, 49, 50, 53, 54, 55, 56, 59) were able to stain the entire contents of subventral gland cells including the gland ducts. Two MABs (MGR 57 and 60) persistently stained only the cut surfaces of the gland cells, and failed to penetrate sufficiently to obtain an even staining.
   Immunofluorescence microscopy was also used to test the cross reactivity of the MAbs with J2 of other species of plant parasitic nematodes (Table 1). It was found that all MAbs also bind to the subventral glands of *G. pallida*, and that all MAbs, except MGR 49, also bind to the subventral glands of *G. tabacum*. In *H. schachtii*, *H. glycines*, *M. incognita* and *M. hapla* no binding to salivary glands was observed, except for MGR 48, which reacted with the subventral glands of *H. glycines*.
Western blotting: The MAbs were tested for reactivity with protein homogenates of J2 which were separated by SDS-PAGE and electroblotted onto PVDF membrane. 11 of the 12 MAbs stained protein bands in the electrophoresis pattern. Together these MAbs identified 4 (6) different polypeptides with apparent molecular weights of about 30-31 (*svp*30; SV-1b), 31-32 (*svp*31a, *svp*31b, *svp*32; SV-1a), 38-39 (*svp*39, SV-2) and 49-50 kDa (*svp*49; SV-3) respectively. All reactive MAbs bound to the SV-2 band, with 10 showing additional binding to one or more of the other bands. MGR 47, 53, 54, 55, and 59 all reacted strongly with the SV-1a, SV-1b and SV-2 kDa bands, while MGR 46, 57 and 60 showed moderate binding to SV-2 and SV-3. Reactivity with all six protein bands was found only with MGR 48. The binding specificity of MGR 56 most resembled that of MGR 48 with the difference that no binding was observed with bands SV-1a and SV-3. Both MGR 48 and MGR 56 stained additional smaller bands in the gel region below SV-1b.
Electron microscopy: Because ultrastructural information about the subventral glands in potato cyst-nematodes was completely lacking, and could only be inferred from studies with related plant-parasitic nematode species (Endo 1984; Endo 1988; Endo 1993), we first examined subventral gland morphology in hatched J2 of *G. rostochiensis*. Thin sections showed that both the cell body and the cell extension are packed with secretory vesicles. It was only in the immediate vicinity of the nucleus that parts of the cytoplasm were free from secretory vesicles. The secretory granules invariably possessed an electron translucent halo which surrounded an electron dense core. This characteristic made it easy to identify the subventral glands in the low-contrast formaldehyde fixed specimens that were used for immunolabelling.
   Immunogold labelling of formaldehyde-fixed J2 with MAb MGR 48 was localised to the subventral salivary glands. Binding of the gold particles occurred both to the electron dense granule matrix and also to the patches of cytoplasm surrounding the granules.

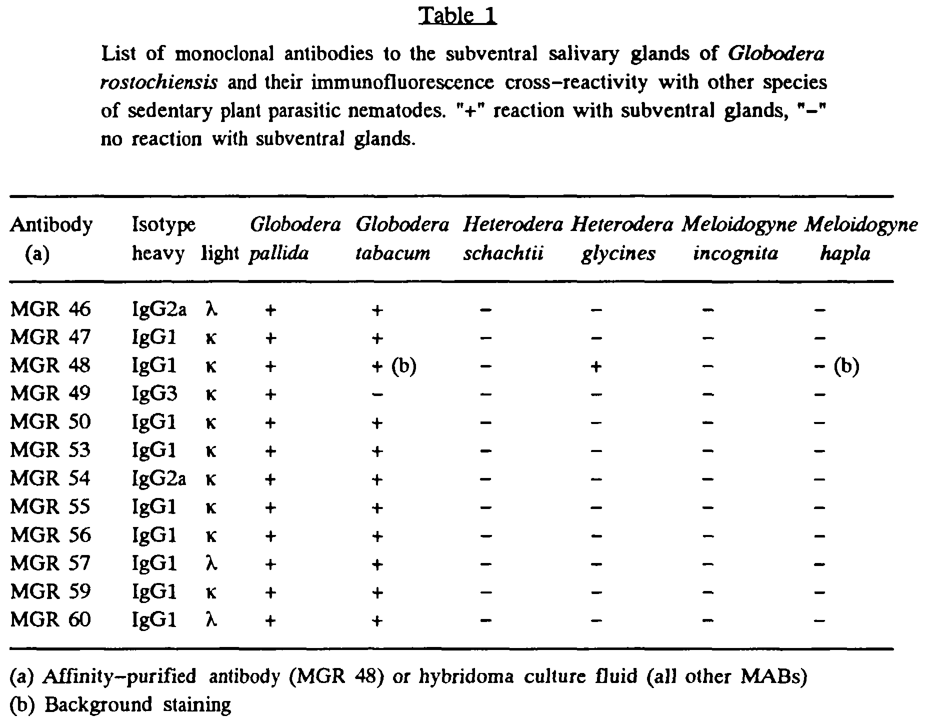

### Induced antigen production

Stylet secretion of J2 of *G. rostochiensis* was induced by incubation with the serotonin antagonist DMT(5-methoxy-N,N-dimethyltryptamine-hydrogen-oxalate; Research Biochemicals Inc., Natick, MA, US), as described previously for preparasitic J2 of *Heterodera glycines* (Goverse et al. 1994). Salivary proteins were collected by sieving to remove the nematodes. Antibody MGR 48 was used for probing Western blots of collected stylet secretions. Secretion of Ag48 (32, 39 and 49 kDa) appeared in samples from DMT and control solutions. At pH below 5.5 the protein pattern varied indicating high sensitivity for acidic hydrolysis. In solutions up to pH 8.0 Ag48 could be detected resulting in a highly stable secretory pattern when the secretions were obtained in standard pore water (0.1 mM H⁺, 0.1 mM K⁺, 0.2 mM Na⁺, 0.35 mM Ca²⁺, 0.3 mM Mg²⁺, 0.3 mM NH₄⁺, 1.7 mM NO₃⁻, and 0.3 mM Cl⁻, pH 6), with ionic concentrations simulating soil fluid. The secretion of Ag48 in control solutions was less for spontaneously hatched J2 than for J2 exposed to potato root diffusate. The enhancing effect of DMT on the secretion of Ag48 was significant. DMT did not harm the nematodes since they were still able to infect potato roots and develop into females as also described for *H. glycines* (Goverse et al, 1994).

### Further embodiments of the invention

1. Antibody directed against a subventral salivary protein of a cyst nematode or at an immunogenic part or equivalent thereof, said protein having a molecular weight of 30-50 kDa and having an isoelectric point (pI) of about 6.8 to 8.7.
2. Antibody as above, wherein said protein is selected from a protein of about 30 kDa having a pI of 7.6, a protein of about 31 kDa having a pI of 7.6, a protein of about 31 kDa having a pI of 7.9, a protein of about 32 kDa having a pI of 6.8, a protein of about 39 kDa having a pI of 7.3, and a protein of about 49 kDa having a pI of 8.7.
3. Antibody as above, wherein said protein comprises an amino acid sequence having a homology of at least 50%, preferably at least 60% with the amino acid sequence depicted in SEQ ID No. 1, 2, 3 or 4.
4. Antibody as above, wherein said cyst nematode belongs to one of the genera *Heterodera*, *Globodera* and *Punctodera*.
5. Antibody as above, wherein said protein is derived from a second-stage juvenile of said nematode.
6. Antibody as above, which comprises one or more variable domains of the complete antibody, such as a single chain antibody.
7. Antibody as above, which comprises a peptide corresponding to an amino acid sequence or encoded by a nucleotide sequence as depicted in SEQ ID No. 5.
8. Polypeptide corresponding to the salivary protein referred to above or to a part thereof, in substantially isolated form.
9. Nucleotide sequence encoding a polypeptide as above.
10. Nucleotide sequence encoding a peptide corresponding to an antibody according as above or a part thereof.
11. Nucleotide as above, comprising the nucleotide sequence 1-336 and/or 391-828 as depicted in SEQ ID No.5.
12. Expression system comprising at least a nucleotide sequence as above and a sequence regulating expression of said nucleotide sequence.
13. Process for protecting a plant against the action of a cyst nematode, wherein the plant is stably transformed using a nucleotide sequence as above or an expression system as above.
14. Transgenic plant containing in its genome a nucleotide sequence as above, and capable of expressing said sequence.

### References (cont.)

Atkinson, H.J., Harris, P.D., Halk, E.J., Novitski, C., Leighton-Sands, J., Nolan, P. & Fox P.C. (1988). Annals of Applied Biology **112**, 459-69.
Bradford, M.M. 1976. Anal. Biochem. **72**, 48-254.
Briggs, M.S. and Gierasch (1986). Adv. Protein Chem. **38**, 109-180.
Cabilly, S. et al. (1984). Proc. Natl. Acad. Sci. USA **81**, 3273-3277.
Clarke, A.J. & Perry, R.N. (1977). Nematologica **23**, 350-68.
Davis, E.L., Aron, L.M., Pratt, L.H. & Hussey, R.S. (1992). Phytopathology **82**, 1244-50.
De Almeida, E.R.P., Gossele, V., Muller C.G., Dockx, J., Reynaerts, A., Botterman, J., Krebbers, E. and Timko, M.P. (1989). Mol. Gen. Genet. **218**, 78-86.
De Boer, J.M., Overmars, H.A., Bakker, J. & Gommers, F.J. (1992a). Parasitology **105**, 461-74.
De Boer, J.M., Overmars, H., Bouwman-Smits, L., De Boevere, M., Gommers, F.J., & Bakker, J. (1992b). Fundamental and Applied Nematology **15**, 495-501.
D'Halluin, K., Bonne, E., Bossut, M., DeBeuckeleer, M., and Leemans, J. (1992). Plant Cell **4**, 1495-1506.
Endo, B.Y. (1984). Proc. Helminthological Soc. Washington **51**, 1-24.
Endo, B.Y. (1987). Journal of Nematology **19**, 469-483.
Endo, B.Y. (1988). Proc. Helminthological Soc. Washington **55**, 117-31.
Firek, S., Draper, J., Owen, M.R.L., Gandecha, A., Cockburn, B., and Whitelam, G.C. (1993). Plant Mol. Biol. **23**, 861-870.
Goding, J.W. (1983). Monoclonal antibodies: principles and practice. London: Academic Press.
Goverse, A., Davis, E.L. and Hussey, R.S. (1994). J. Nematology **26**, 251-259.
Guerche, P., Tire, C., Grossi De Sa, F., De Clercq, A., Van Montagu, and Krebbers, E. (1990). Plant Cell **2**, 69-478.
Hendriks, T., Vreugdenhil, D., Stiekema, W.J. (1991). Plant Mol. Biol., 385-394.
Horsch, R.B., Fry, J.E., Hoffmann, N.L., Eichholtz, N.L., Rogers, S.G., and Fraley, R.T. (1985). Science **227**, 1229-1231.
Hussey, R.S. (1989a). Ann. Rev. Phytopathol. **27**, 123-41.
Hussey, R.S. (1989b). J. Nematol. **21**, 392-98.
Hussey, R.S. & Mims, C.W. (1991). Protoplasma **162**, 99-107.
Kabat, E.A., Wu, T.T, Perry, H.M., Gottesman, K.S. and Foeller, C. (1991). Sequences of proteins of immunological interest, 5th Ed. US Dept. of Health and Human Services, p.9.
Kaplan, D.T. & Davis, E.L. (1987). In Vistas on Nematology (ed. Veech, J.A. & Dickson, D.W.), pp. 267-76. Haittsville: Society of Nematologists.
Kay, R., Chan, A., Daly, M., and McPherson, J. (1987). Science **236**, 1299-1302.
Langridge, W.H.R., Fitzgerald, K.J., Koncz, C., Schell, J., and Szalay, A.A. (1994). Proc. Natl. Acad. Sci. USA **86**, 3219-3223.
Logemann, J., Lipphardt, S., Lorz, H., Hauser, I., Willmitzer, L., and Schell, J. (1989). Plant Cell **1**, 151-158.
Nap, J.P., van Spanje, M., Dirkse, W.G., Baarda, G., Mlynarova, L., Loonen, A., Grondhuis, P. and Stiekema, W.J. (1993). Plant Mol. Biol. **23**, 605-612.
Nap, J.P., Dirkse, W.G., Louwerse. J., Onstenk, J., Visser, R., Loonen, A., Heidekamp, F. and Stiekema, W.J. (1992). Plant Mol. Biol. **20**, 683-694.
Neuhoff, V., Arold, N., Taube, D. and Ehrhardt, W. 1988. Electrophoresis 9, 255-262.
O'Farrell, P.H. 1975. Journal of Biological Chemistry **250**, 4007-21.
Rumpenhorst, H.J. (1984). Nematologica **30**, 77-85.
Schots, A., De Boer, J., Schouten, A., Roosien, J., Zilverentant, J.F., Pomp, H., Bouwman-Smits, L., Overmars, H., Gommers, F.J., Visser, B., Stiekema, W.J., & Bakker, J. (1992a). Neth. J. Plant Pathol. **98**, Supplement 2, 183-91.
Schots, A., Pomp, R. & Van Muiswinkel, W.B. (1992b). Production of Monoclonal Antibodies. In: Techniques in Fish Immunology (ed. Stolen et al), pp. 1-18. Fair Haven: SOS Publications.
Shirasu, Y. et al. (1988). Hybridoma **7**, 485-493.
Spurr, A. R. (1969). J. Ultrastr. Res. **26**, 31-43.
Vasil, V., Castillo, A.M., Fromm, M.E., and Vasil, I.K. (1992). Biotechnology **10**, 667-674.
Williams, S. Friedrich, L., Dincher, S., Carozzi, N., Kessmann, H., Ward, E., and Ryals, J. (1992). Biotechnology **10**, 540-543.
Wyss, U. & Zunke, U. (1986). Rev. Nématol. **9**, 153-65.
Zuckerman, B.M. & Jansson, H.-B. (1984). Ann. Rev. Phytopathology **22**, 95-113.

## Claims

1. Peptide having cellulase activity and exhibiting at least 40% amino acid identity in the primary structure with the amino acid sequence shown in one of SEQ ID NO.'s 1, 2, 3 and 4 or comprising a series of at least 7 contiguous amino acids of the amino acid sequence shown in one of SEQ ID NO.'s 1, 2, 3 and 4.

2. Peptide according to claim 1, wherein said amino acid identity in the primary structure is at least 50%, or said series comprises at least 8 contiguous amino acids of the amino acid sequence shown in one of SEQ ID NO.'s 1, 2, 3 and 4.

3. Peptide according to claim 1, wherein said amino acid identity in the primary structure is at least 60%, or said series comprises at least 10 contiguous amino acids of the amino acid sequence shown in one of SEQ ID NO.'s 1, 2, 3 and 4.

4. Peptide according to any one of claims 1-3, wherein said peptide corresponds to a nematodal peptide or part thereof.

5. Nucleotide sequence encoding a peptide according to any one of claims 1-4.

6. Method for producing a peptide having cellulase activity comprising translating a nucleotide sequence according to claim 5, in a manner known per se for translating nucleotide sequences.

7. Expression vector containing a nucleotide sequence according to claim 5.

8. Recombinant host cell containing, in addition to its genomic nucleic acid, a nucleotide sequence according to claim 5.

9. Host cell according to claim 8, also capable of expressing an amylase, xylanase, glucanase, oxidoreductase, α-glucuronidase, lipase, esterase, or protease.

10. Enzyme preparation containing an effective amount of a cellulolytic peptide according to one of claims 1-4.

11. Antibody directed at a peptide according to any one of claims 1-4.

12. Use of a peptide according to any one of claims 1-4 or an antibody according to claim 13 in the protection of plants against parasitic nematodes.

13. Use of a peptide according to any one of claims 1-4 or of an enzyme preparation according to claim 10 in the food and beverage industry, in paper or clothing industry or in waste treatment.


SEQUENCE LISTING

(1) GENERAL INFORMATION:

(i) APPLICANT:
(A) NAME: Rijkslandbouwuniversiteit Wageningen
(B) STREET: P.O. Box 9101
(C) CITY: Wageningen
(E) COUNTRY: The Netherlands
(F) POSTAL CODE (ZIP): 6700 HB
(G) TELEPHONE: 0317-489111

(ii) TITLE OF INVENTION: Novel cellulases

(iii) NUMBER OF SEQUENCES: 7

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP (96.07.08)

(2) INFORMATION FOR SEQ ID NO: 1:

(i) SEQUENCE CHARACTERISTICS:
(A) LENGTH: 1080 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(v) FRAGMENT TYPE: internal

(vi) ORIGINAL SOURCE:
(A) ORGANISM: Heterodera glycines

(ix) FEATURE:
(A) NAME/KEY: CDS
(B) LOCATION: 2..910

(xi) SEQUENCE DESCRIPTION: SEQ ID NO: 1:

T GCG GTG GCG CCG CCG TTC GGG CAA TTG TCC GTC AAA GGC ACC AAT 46
Ala Val Ala Pro Pro Phe Gly Gln Leu Ser Val Lys Gly Thr Asn
1               5                   10                  15

TTG GTC GGC TCA AAC GGC AAA CCG GTG CAG CTG ATC GGC AAT TCG TTG 94
Leu Val Gly Ser Asn Gly Lys Pro Val Gln Leu Ile Gly Asn Ser Leu
                20                  25                  30

TTC TGG CAC CAG TGG TAC CCA CAA TTT TGG AAT GCC CAA ACA GTG AAG 142
Phe Trp His Gln Trp Tyr Pro Gln Phe Trp Asn Ala Gln Thr Val Lys
            35                  40                  45

GCA CTC AAA TGC AAT TGG AAT TCC AAT GTC GTG CGC ACC GCA ATG GGC 190
Ala Leu Lys Cys Asn Trp Asn Ser Asn Val Val Arg Thr Ala Met Gly
        50                  55                  60

GTG GAA CAG GGC GGC TAT CTG AGT GAC GCG AAC ACC GCC TAC CGA CTG 238
Val Glu Gln Gly Gly Tyr Leu Ser Asp Ala Asn Thr Ala Tyr Arg Leu
    65                  70                  75

ACG GCA GCT GTG ATT GAG GCG GCC ATT GCA CAG GGC ATT TAC GTG ATC 286
Thr Ala Ala Val Ile Glu Ala Ala Ile Ala Gln Gly Ile Tyr Val Ile
80                  85                  90                  95

GTC GAT TGG CAC GCG CAT GAG CCG AAC GCG GAC AAG GCG ATT GAA TTC 334
Val Asp Trp His Ala His Glu Pro Asn Ala Asp Lys Ala Ile Glu Phe
                100                 105                 110

TTC ACC AAA ATT GCG AAA GCG TAC GGC TCC AAC CCT CAC TTG CTT TAC 382
Phe Thr Lys Ile Ala Lys Ala Tyr Gly Ser Asn Pro His Leu Leu Tyr
            115                 120                 125

GAA ACG TTT AAC GAG CCA TTG GAT GTG TCT TGG AAC GAT GTG CTT GTC 430
Glu Thr Phe Asn Glu Pro Leu Asp Val Ser Trp Asn Asp Val Leu Val
        130                 135                 140

CCT TAC CAT AAA AAG GTT ATT TCT GCA ATT CGT GCC ATC GAC AAA AAG 478
Pro Tyr His Lys Lys Val Ile Ser Ala Ile Arg Ala Ile Asp Lys Lys
    145                 150                 155

AAT GTG ATC ATT CTC GGC ACT CCC AAA TGG TCT CAA GAT GTT GAC GTG 526
Asn Val Ile Ile Leu Gly Thr Pro Lys Trp Ser Gln Asp Val Asp Val
160                 165                 170                 175

GCG GCC CAA AAT CCG ATC AAA GGA TTC AGT AAT TTG ATG TAC ACT CTC 574
Ala Ala Gln Asn Pro Ile Lys Gly Phe Ser Asn Leu Met Tyr Thr Leu
                180                 185                 190

CAC TTC TAT GCG TCC AGT CAC TTT GTT GAT GGA CTT GGC AAT AAG CTT 622
His Phe Tyr Ala Ser Ser His Phe Val Asp Gly Leu Gly Asn Lys Leu
            195                 200                 205

AAG ACC GCC GTA AAT AAG GGT CTT CCG GTG TTC GTC ACT GAG TAC GGT 670
Lys Thr Ala Val Asn Lys Gly Leu Pro Val Phe Val Thr Glu Tyr Gly
        210                 215                 220

ACA TGC GAA GCG TCT AGC AAC GGT AAT CTG AAC TCC GGC TCA ATG TCA 718
Thr Cys Glu Ala Ser Ser Asn Gly Asn Leu Asn Ser Gly Ser Met Ser
    225                 230                 235

AGC TGG TGG AGC CTG TTG GAC CAA CTG AAA ATT TCG TAC GTC AAT TGG 766
Ser Trp Trp Ser Leu Leu Asp Gln Leu Lys Ile Ser Tyr Val Asn Trp
240                 245                 250                 255

TCA ATC ACT GAC AAA AGC GAA GCT TGT GCA GCG CTC ACT GGC GGA ACA 814
Ser Ile Thr Asp Lys Ser Glu Ala Cys Ala Ala Leu Thr Gly Gly Thr
                260                 265                 270

TCG GCT GCC AAT GTT GGC ACT TCC TCC CGC TGG ACG CAG TCT GGC AAT 862
Ser Ala Ala Asn Val Gly Thr Ser Ser Arg Trp Thr Gln Ser Gly Asn
            275                 280                 285

ATG GTA GCT TCG CAA CAC AAG AAA AAA TCC ACC GGT GTG AAA TGC TAAATAAAGT 917
Met Val Ala Ser Gln His Lys Lys Lys Ser Thr Gly Val Lys Cys
        290                 295                 300

CTGAAGTCCG GATGATCAAC TGAAACTGAA GAAAATGGAA AATGTGAAGA TTGCGAAAAA 977

ATAAATATAT ATAAAATTTG CATGCGAATG TTTATCGAAA TTCCGATTAA ATTGTTTTTT 1037

GAGCGCAGAT AAATCGCAAA GCATAAAAAA AAAAAAAAAA AAA 1080

(3) INFORMATION FOR SEQ ID NO: 2:

(i) SEQUENCE CHARACTERISTICS:
(A) LENGTH: 1511 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(v) FRAGMENT TYPE: internal

(vi) ORIGINAL SOURCE:
(A) ORGANISM: Heterodera glycines

(ix) FEATURE:
(A) NAME/KEY: CDS
(B) LOCATION: 2..1456

(xi) SEQUENCE DESCRIPTION: SEQ ID NO: 2:

G GTG GCG CCG CCG TTT GGG CAA TTG TCC GTT TCC GGC ACC AAT TTG 46
Val Ala Pro Pro Phe Gly Gln Leu Ser Val Ser Gly Thr Asn Leu
1               5                   10                  15

GTC GGC GCC AAC GGA CAA CCC GTA CAG CTG ATC GGC AAC TCA CTG TTC 94
Val Gly Ala Asn Gly Gln Pro Val Gln Leu Ile Gly Asn Ser Leu Phe
                20                  25                  30

TGG CAC CAA TGG TAC CCG CAG TTT TGG AAC GCT GAC ACA GTG AAG GCA 142
Trp His Gln Trp Tyr Pro Gln Phe Trp Asn Ala Asp Thr Val Lys Ala
            35                  40                  45

CTC AAA TGC AAT TGG AAT GCC AAT GTC ATC CGG GGG GCC ATG GGC GTG 190
Leu Lys Cys Asn Trp Asn Ala Asn Val Ile Arg Gly Ala Met Gly Val
        50                  55                  60

GAC GAG GGC GGC TAT CTG AGT GAC GCG AAC ACG GCT TAC AAT CTG ATG 238
Asp Glu Gly Gly Tyr Leu Ser Asp Ala Asn Thr Ala Tyr Asn Leu Met
    65                  70                  75

GTG GCA GTG ATC GAA GCG GCC ATT TCC AAT GGC ATT TAC GTG ATC GTC 286
Val Ala Val Ile Glu Ala Ala Ile Ser Asn Gly Ile Tyr Val Ile Val
80                  85                  90                  95

GAT TGG CAT GCC CAC AAT TCA CAT CCG GAC GAA GCG GTC AAA TTC TTC 334
Asp Trp His Ala His Asn Ser His Pro Asp Glu Ala Val Lys Phe Phe
                100                 105                 110

ACC CGA ATT GCT CAA GCG TAC GGC TCC TAC CCT CAC ATT TTG TAC GAG 382
Thr Arg Ile Ala Gln Ala Tyr Gly Ser Tyr Pro His Ile Leu Tyr Glu
            115                 120                 125

GAT TTC AAC GAG CCG CTG AGC GTT TCG TGG ACC GAT GTG CTG GTG CCA 430
Asp Phe Asn Glu Pro Leu Ser Val Ser Trp Thr Asp Val Leu Val Pro
        130                 135                 140

TAC CAC AAA AAG GTC ATT GCT GCC ATC CGA GCT ATT GAC AAG AAA AAT 478
Tyr His Lys Lys Val Ile Ala Ala Ile Arg Ala Ile Asp Lys Lys Asn
    145                 150                 155

GTG ATC ATT CTC GGC ACT CCA ACA TGG TCC CAA GAC GTG GAT GTG GCA 526
Val Ile Ile Leu Gly Thr Pro Thr Trp Ser Gln Asp Val Asp Val Ala
160                 165                 170                 175

TCA CAG AAC CCA ATC AAA GAC TAC CAA AAT CTG ATG TAC ACT CTC CAC 574
Ser Gln Asn Pro Ile Lys Asp Tyr Gln Asn Leu Met Tyr Thr Leu His
                180                 185                 190

TTT TAC GCG TCC AGT CAC TTC ACG AAT GAT CTT GGT GCC AAG CTC AAA 622
Phe Tyr Ala Ser Ser His Phe Thr Asn Asp Leu Gly Ala Lys Leu Lys
            195                 200                 205

ACA GCC GTG AAC AAC GGT TTG CCT GTG TTC GTC ACT GAG TAC GGC ACA 670
Thr Ala Val Asn Asn Gly Leu Pro Val Phe Val Thr Glu Tyr Gly Thr
        210                 215                 220

TGC GAA GCG TCG GGC AAC GGC AAC TTG AAC ACT GAC TCG ATG TCC AGC 718
Cys Glu Ala Ser Gly Asn Gly Asn Leu Asn Thr Asp Ser Met Ser Ser
    225                 230                 235

TGG TGG ACT CTG CTG GAC AGC TTG AAG ATT TCA TAC GCC AAC TGG GCA 766
Trp Trp Thr Leu Leu Asp Ser Leu Lys Ile Ser Tyr Ala Asn Trp Ala
240                 245                 250                 255

ATC TCC GAC AAA AGT GAG GCC TGC TCA GCA CTG AGC CCC GGT ACA ACT 814
Ile Ser Asp Lys Ser Glu Ala Cys Ser Ala Leu Ser Pro Gly Thr Thr
                260                 265                 270

GCT GCC AAT GTC GGT GTT TCG TCC CGT TGG ACA TCC TCC GGA AAT ATG 862
Ala Ala Asn Val Gly Val Ser Ser Arg Trp Thr Ser Ser Gly Asn Met
            275                 280                 285

GTT GCT TCG TAC TAC AAG AAA AAA TCC ACC GGC GTA AGC TGT AGC GGC 910
Val Ala Ser Tyr Tyr Lys Lys Lys Ser Thr Gly Val Ser Cys Ser Gly
        290                 295                 300

TCA AGT TCC GGC AGC TCT TCG GGC TCG AGT TCT GGC TCT TCC GGT TCA 958
Ser Ser Ser Gly Ser Ser Ser Gly Ser Ser Ser Gly Ser Ser Gly Ser
    305                 310                 315

AGT TCC GGC AGC TCT TCC GGC TCA AGT TCC GGC AGC TCT TCC GGC TCA 1006
Ser Ser Gly Ser Ser Ser Gly Ser Ser Ser Gly Ser Ser Ser Gly Ser
320                 325                 330                 335

AGT TCG GGC TCC TCT GGT TCG AGC TCA GGG TCC AGC TCG GGT TCG GGA 1054
Ser Ser Gly Ser Ser Gly Ser Ser Ser Gly Ser Ser Ser Gly Ser Gly
                340                 345                 350

TCC GCC AGC ATC TCT GTA GTC CCA TCC AAC ACG TGG AAT GGC GGT GGT 1102
Ser Ala Ser Ile Ser Val Val Pro Ser Asn Thr Trp Asn Gly Gly Gly
            355                 360                 365

CAG GTC AAC TTC GAA ATC AAA AAC ATC GGG TCC GTG CCA TTG TGT GGC 1150
Gln Val Asn Phe Glu Ile Lys Asn Ile Gly Ser Val Pro Leu Cys Gly
        370                 375                 380

GTT GTG TTC AGC GTC TCT CTT CCC TCA GGG ACC ACG CTT GGT GGA TCG 1198
Val Val Phe Ser Val Ser Leu Pro Ser Gly Thr Thr Leu Gly Gly Ser
    385                 390                 395

TGG AAC ATG GAA TCC GCA GGC TCC GGC CAA TAC AGC TTG CCA AGT TGG 1246
Trp Asn Met Glu Ser Ala Gly Ser Gly Gln Tyr Ser Leu Pro Ser Trp
400                 405                 410                 415

GTC AGA ATT GAG GCC GGA AAA TCG AGC AAA GAC GCG GGG CTG ACA TTC 1294
Val Arg Ile Glu Ala Gly Lys Ser Ser Lys Asp Ala Gly Leu Thr Phe
                420                 425                 430

AAC GGA AAA GAT AAG CCA ACG GCG AAA AAT TGT GAC GAC GAA GAA ATG 1342
Asn Gly Lys Asp Lys Pro Thr Ala Lys Asn Cys Asp Asp Glu Glu Met
            435                 440                 445

TTA GGG AAA AGA CAA AGC GCA AAA TGG GAG GAA AAG AAT GGG AAA ATG 1390
Leu Gly Lys Arg Gln Ser Ala Lys Trp Glu Glu Lys Asn Gly Lys Met
        450                 455                 460

AAA GAG TGG AGA AAA AAG AAC AAA GGG AAA GAA AGA CAA AGA ATT AAT 1438
Lys Glu Trp Arg Lys Lys Asn Lys Gly Lys Glu Arg Gln Arg Ile Asn
    465                 470                 475

GAT TAT GAT AAG AGA TAATAACTGA TAAAGTTTAG CATAAAATGT TGACCAAAGT 1493
Asp Tyr Asp Lys Arg
480 485

AAAAAAAAAA AAAAAAAA 1511

(4) INFORMATION FOR SEQ ID NO: 3:

(i) SEQUENCE CHARACTERISTICS:
(A) LENGTH: 106 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Globodera rostochiensis

(ix) FEATURE:
(A) NAME/KEY: Modified-site
(B) LOCATION: 30
(D) OTHER INFORMATION: "Asp may be replaced by Val"

(xi) SEQUENCE DESCRIPTION: SEQ ID NO: 3:

Met Pro Thr Ala Thr Pro Pro Pro Tyr Gly Gln Leu Ser Val Ser Gly
1               5                   10                  15

Thr Lys Leu Val Gly Ser Asn Gly Gln Pro Val Gln Leu Asp Gly Asn
            20                  25                  30

Ser Leu Phe Trp His Gln Phe Gln Gly Gln Tyr Trp Asn Ala Asp Thr
        35                  40                  45

Val Lys Ala Leu Lys Cys Asn Trp Asn Ala Asn Val Val Arg Ala Ala
    50                  55                  60

Val Gly Val Asp Leu Glu Arg Gly Tyr Met Ser Asp Pro Thr Ser Ala
65                  70                  75                  80

Tyr Asn Leu Ala Val Ala Val Ile Glu Ala Ala Ile Ser Gln Gly Met
                85                  90                  95

Tyr Val Ile Val Asp Trp His Ser Thr Glu
            100                 105

(5) INFORMATION FOR SEQ ID NO: 4:

(i) SEQUENCE CHARACTERISTICS:
(A) LENGTH: 26 amino acids
(B) TYPE: amino acid
(C) STRANDEDNESS: single
(D) TOPOLOGY: linear

(ii) MOLECULE TYPE: peptide

(iii) HYPOTHETICAL: NO

(vi) ORIGINAL SOURCE:
(A) ORGANISM: Globodera rostochiensis

(xi) SEQUENCE DESCRIPTION: SEQ ID NO: 4:

Val Thr Ala Pro Pro Tyr Gly Pro Leu Ala Val Asn Gly Lys Phe Leu
1               5                   10                  15

Val Gln Lys Ser Thr Lys Gln Thr Val Asp
            20                  25

(6) INFORMATION FOR SEQ ID NO: 5:

(i) SEQUENCE CHARACTERISTICS
(A) LENGTH: 819 nucleotides
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Mouse (Mus musculus)

(ix) FEATURE:
(A) KEY: Antibody variable domain light chain
(B) LOCATION: 1-336

(ix) FEATURE:
(A) KEY: Complementarity Determining Region
(B) LOCATION: 73-117

(ix) FEATURE:
(A) KEY: Complementarity Determining Region
(B) LOCATION: 163-183

(ix) FEATURE:
(A) KEY: Complementarity Determining Region
(B) LOCATION: 280-306

(ix) FEATURE:
(A) KEY: Gly-Ser linker
(B) LOCATION: 337-381

(ix) FEATURE:
(A) KEY: Antibody variable domain heavy chain
(B) LOCATION: 382-819

(ix) FEATURE:
(A) KEY: Complementarity Determining Region
(B) LOCATION: 472-486

(ix) FEATURE:
(A) KEY: Complementarity Determining Region
(B) LOCATION: 529-579

(ix) FEATURE:
(A) KEY: Complementarity Determining Region
(B) LOCATION: 676-696

(xi) SEQUENCE DESCRIPTION: SEQ ID NO: 5:

GAT GTT GTG ATG ACC CAA ACT CCA CTC ACT TTG TCG GTT ACC ATT GGA 48
Asp Val Val Met Thr Gln Thr Pro Leu Thr Leu Ser Val Thr Ile Gly
                5                   10                  15

CAA CCA GCC TCC ATC TCT TGC AAG TCC AGT CAG AGC CTC TTA CAT AGT 96
Gln Pro Ala Ser Ile Ser Cys Lys Ser Ser Gln Ser Leu Leu His Ser
            20                  25                  30

GAT GGA AAG ACA TAT TTG AGT TGG TTG TCA CAG AGG CCA GGC CAG TCG 144
Asp Gly Lys Thr Tyr Leu Ser Trp Leu Ser Gln Arg Pro Gly Gln Ser
        35                  40                  45

CCA AAG CGC CTT ATC TAT CTG GTG TCT AAA CTG GAC TCT GGA GTC CCT 192
Pro Lys Arg Leu Ile Tyr Leu Val Ser Lys Leu Asp Ser Gly Val Pro
    50                  55                  60

GAC AGG TTC ACT GGC AGT GGA TCA GGG ACA GAT TTC ACA CTG AAA ATC 240
Asp Arg Phe Thr Gly Ser Gly Ser Gly Thr Asp Phe Thr Leu Lys Ile
65                  70                  75                  80

AGC AGA GTG GAG GCT GTG GAT TTG GGA GTT TAT TAT TGT TGG CAA GGA 288
Ser Arg Val Glu Ala Val Asp Leu Gly Val Tyr Tyr Cys Trp Gln Gly
                85                  90                  95

ACA CAT CTT CCG TAC ACG TTC GGA GGG GGG ACC AAG CTG GAA ATA AAA 336
Thr His Leu Pro Tyr Thr Phe Gly Gly Gly Thr Lys Leu Glu Ile Lys
            100                 105                 110

GGA GGT GGT GGT TCT GGA GGT GGA GGA TCT GGT GGA GGC GGA TCC GAG 384
Gly Gly Gly Gly Ser Gly Gly Gly Gly Ser Gly Gly Gly Gly Ser Glu
        115                 120                 125

GTT CAG CTG CAG CAG TCT GGG CCT GAG CTC GTG AGG CCT GGG GCT TCA 432
Val Gln Leu Gln Gln Ser Gly Pro Glu Leu Val Arg Pro Gly Ala Ser
    130                 135                 140

GTG AAG ATG TCC TGC AAG GCT TCA GGC TAT ACC TTC ACC GAC TAT TGG 480
Val Lys Met Ser Cys Lys Ala Ser Gly Tyr Thr Phe Thr Asp Tyr Trp
145                 150                 155                 160

ATG CAC TGG GTG AAA CAG AGG CCT GGA CAA GGC CTT GAG TGG ATT GGC 528
Met His Trp Val Lys Gln Arg Pro Gly Gln Gly Leu Glu Trp Ile Gly
                165                 170                 175

ATG ATT GAT CCT TCC AAT GGT GGG ACT TGG TTA AAT CAG CAG TTC AAG 576
Met Ile Asp Pro Ser Asn Gly Gly Thr Trp Leu Asn Gln Gln Phe Lys
            180                 185                 190

GGC AAA GCA ACA TTG AAT GTG GCC AAA TCC TCC AAC ACA GCC TAC ATG 624
Gly Lys Ala Thr Leu Asn Val Ala Lys Ser Ser Asn Thr Ala Tyr Met
        195                 200                 205

CAG CTC AGC AGC CTG ACG TCT GAG GAC TCT GCA GTC TAT TAC TGT TCA 672
Gln Leu Ser Ser Leu Thr Ser Glu Asp Ser Ala Val Tyr Tyr Cys Ser
    210                 215                 220

AGA AAC TCC CCC TGG TTT GCT TAC TGG GGC CAA GGG ACT CTG GTC ACA 720
Arg Asn Ser Pro Trp Phe Ala Tyr Trp Gly Gln Gly Thr Leu Val Thr
225                 230                 235                 240

GTC TCT GCA GCC AAA ACG ACA CCC CCA TCG GTC TAT CCA CTG GTT GGG 768
Val Ser Ala Ala Lys Thr Thr Pro Pro Ser Val Tyr Pro Leu Val Gly
                245                 250                 255

GCG GCC GCA GAA CAA AAA CTC ATC TCA GAA GAG GAT CTG AAT GAT ATC 816
Ala Ala Ala Glu Gln Lys Leu Ile Ser Glu Glu Asp Leu Asn Asp Ile
            260                 265                 270

TAG                                                                    819
 -

(7) INFORMATION FOR SEQ ID NO: 6:

  (i) SEQUENCE CHARACTERISTICS
       (A) LENGTH: 48 nucleotides
       (B) TYPE: nucleic acid
       (C) STRANDEDNESS: single
       (D) TOPOLOGY: linear

  (ii) MOLECULE TYPE: cDNA to mRNA

  (ix) FEATURE:
       (A) KEY: linker peptide 202
       (B) LOCATION: 1-48

  (xi) SEQUENCE DESCRIPTION: SEQ ID NO: 6:

CTC GAG GGT AAA TCC TCA GGA TCC GGC TCC GAA TCC AAA CTC GAG TCT    48
Leu Glu Gly Lys Ser Ser Gly Ser Gly Ser Glu Ser Lys Leu Glu Ser
                 5                  10                  15

(8) INFORMATION FOR SEQ ID NO: 7:

  (i) SEQUENCE CHARACTERISTICS
       (A) LENGTH: 72 nucleotides
       (B) TYPE: nucleic acid
       (C) STRANDEDNESS: single
       (D) TOPOLOGY: linear

  (ii) MOLECULE TYPE: cDNA to mRNA

  (vi) ORIGINAL SOURCE:
       (A) ORGANISM: Mouse (Mus musculus)

  (ix) FEATURE:
       (A) KEY: Signal peptide
       (B) LOCATION: 1-72

  (xi) SEQUENCE DESCRIPTION: SEQ ID NO: 7:

ATG GGC ATC AAG ATG GAG ACA CAT TCT CAG GTC TTT GTA TAC ATG TTG    48
Met Gly Ile Lys Met Glu Thr His Ser Gln Val Phe Val Tyr Met Leu
                 5                  10                  15

CTG TGG TTG TCT GGT GTC GAC GGT                                    72
Leu Trp Leu Ser Gly Val Asp Gly
             20